# EUROPEAN PATENT APPLICATION

(11) **EP 4 421 090 A1**
(43) Date of publication of application: **28.08.2024**
(21) Application number: 22883933.8
(22) Date of filing: 17.10.2022
(51) Int. Cl.: C07K 16/00, A61P 35/00, C07K 16/28, A61K 39/00

(54) **FC VARIANTS HAVING IMPROVED PH-DEPENDENT FCRN BINDING CAPACITY AND FCGAMMA-RIIIA BINDING SELECTIVITY**

(30) Priority: 18.10.2021 KR 20210138530; 18.10.2021 KR 20210138763
(71) Applicant: Korea University Research and Business Foundation, Seoul 02841 (KR)
(72) Inventor: JUNG, Sang Taek, Seoul 06217 (KR); KYUNG, Munsu, Gimpo-si, Gyeonggi-do 10109 (KR); KO, Sanghwan, Seoul 02709 (KR); JO, Migyeong, Seoul 02812 (KR); KIM, Suyeon, Seoul 07211 (KR); KO, Woo Hyung, Seoul 08018 (KR)
(74) Representative: Isarpatent
(86) International application number: PCT/KR2022/015765
(87) International publication number: WO 2023/068710

(57) **Abstract**

The present invention relates to Fc variants which have improved half-life by binding to and unbinding from FcRn in a pH-dependent manner, and which have improved capacity to selectively bind to Fcy receptors. Novel human Fc domain variants of the present invention have lower capacity to bind to immune-inhibiting receptor FcyRIIb and have higher capacity to bind to immune activating receptor FcyRIIIa (increased A/I ratio) than a wild-type human antibody Fc domain and conventional antibodies approved as antibody therapeutic agents, thereby having remarkably improved ADCC induction ability and having maximized half-life in blood in which excellent pH-selective FcRn binding and unbinding capacity is exhibited, and thus bind to numerous peptide drug therapeutics having a low half-life and retention time in the body so as to enable the peptide drug therapeutics to have an increased blood half-life and exhibit long-term drug efficacy, and can maximize the immune mechanism of therapeutic protein drugs so as to be effectively used as an improved antibody drug.

## Description

### [Technical Field]

The present invention relates to Fc variants which have improved half-life by binding to and unbinding from FcRn in a pH-dependent manner, and which have improved capacity to selectively bind to Fc gamma receptors.

### [Background Art]

Protein therapeutic agents have very high specificity for disease targets and low side effects and toxicity and thus are widely used in clinical trials by rapidly replacing non-specific small molecule compound therapeutic agents, and among protein therapeutic agents currently used in clinical trials, antibody therapeutic agents and Fc-fusion protein therapeutic agents that are fused with the Fc region of an antibody make up the largest part. An antibody provides a linkage between the humoral and cellular immune systems, and a Fab region of the antibody recognizes an antigen, whereas an Fc domain region binds to a receptor (Fc receptor or FcR) for an antibody (immunoglobulin) on a cell that is differentially expressed by all immunocompetent cells. An Fc receptor binding site on the Fc region of the antibody binds to a cell through the Fc region by binding to the Fc receptor (FcR) on the cell and the antibody binds to the Fc receptor on the cell surface to trigger a variety of important biological responses, including control of phagocytosis and destruction of antibody-coated particles, removal of immune complexes, lysis of antibody-coated target cells by killing cells (antibody-dependent cell-mediated cytotoxicity, or ADCC), release of inflammatory mediators, placental transfer and immunoglobulin production (Deo, Y.M. et al., Immunol. Today 18(3):127-135 (1997)). As such, the Fc domain plays a critical role in the collection of immune cells and ADCC and antibody dependent cellular phagocytosis (ADCP). In particular, the ADCC and ADCP functions, which are the effector functions of the antibody, depend on interaction with Fc receptors present on the surfaces of many cells. Human Fc receptors are classified into five types, and the type of immune cell to be collected is determined depending on which Fc receptor the antibody binds to. Accordingly, attempts to modify antibodies to collect specific cells may be very important in the field of treatment.

Therapeutic antibodies show very high specificity to targets compared to conventional small molecule drugs, have not only low biotoxicity and few side effects, but also an excellent blood half-life of about 3 weeks, and thus are considered as one of the most effective cancer therapy methods. In fact, large pharmaceutical companies and research institutes around the world are accelerating the research and development of therapeutic antibodies that specifically bind to cancer cells, including cancer-causing factors, to effectively remove the cancer cells. Companies for developing therapeutic antibody drugs mainly consist of pharmaceutical companies such as Roche, Amgen, Johnson & Johnson, Abbott, and BMS. Particularly, Roche includes representative products of Herceptin, Avastin, Rituxan, and the like for anticancer treatment, and these three therapeutic antibodies not only achieved large profits, achieving sales of approximately $19.5 billion in the global market in 2012, but are also leading the antibody drug market of the world. Johnson & Johnson, which developed Remicade, is also growing rapidly in the global antibody market due to increased sales, and pharmaceutical companies such as Abbott and BMS are also known to have many therapeutic antibodies in the final stages of development. As a result, biopharmaceuticals containing therapeutic antibodies that are specific for disease targets and have low side effects are rapidly replaced in the global pharmaceutical market, where small molecule drugs had the initiative.

It has been reported that the half-life and persistence of immunoglobulins (antibodies) in the blood/in vivo are largely dependent on the binding of Fc to a neonatal Fc receptor (FcRn), one of IgG binding ligands. It has been reported that the FcRn is mainly expressed in endothelial cells and epithelial cells, and binds to the CH₂-CH₃ boundary region of the antibody Fc region to maintain homeostasis of antibody concentration in the body and increase the half-life of antibodies in the blood through a recycling process to move into the cells and then be released into the plasma. Specifically, the Fc fragment of immunoglobulin is taken up by endothelial cells through non-specific cellular uptake and then introduced into acidic endosomes. The FcRn binds to immunoglobulins under acidic pH (< 6.5) in endosomes and releases the immunoglobulins under basic pH (> 7.4) in the bloodstream. Therefore, the FcRn recovers the immunoglobulins from the lysosomal degradation pathway. When the serum immunoglobulin level decreases, the amount of immunoglobulins may be increased by using more FcRn molecules for immunoglobulin binding. On the contrary, when the serum immunoglobulin level increases, the FcRn is saturated to increase the proportion of cellularly taken immunoglobulins to be degraded (Ghetie and Ward, Annu. Rev. Immunol. 18: 739-766, 2000). In other words, the blood half-life and persistence of the antibody largely depend on the binding between the Fc region of the antibody and FcRn (neonatal Fc receptor) as one of the IgG binding ligands. The Fc region of the antibody, which is responsible for collecting immune leukocytes or serum complement molecules so as to eliminate damaged cells such as cancer cells or infected cells, is a region between the Cγ2 and Cγ3 domains, and mediates interaction with the neonatal receptor FcRn, whose binding recycles endocytosed antibodies from endosomes to the bloodstream (Raghavan et al., 1996, Annu Rev Cell Dev Biol 12: 181-220; Ghetie et al., 2000, Annu Rev Immunol 18: 739-766). This process is caused by the large size of the full-length molecule and associated with the inhibition of kidney filtration to have a favorable antibody serum half-life in the range of 1 to 3 weeks. In addition, the binding of Fc to FcRn also plays an important role in antibody transport. Therefore, the Fc region plays an essential role in maintaining prolonged serum persistence by circulating antibodies through intracellular trafficking and recycling mechanisms, and thus in order to improve the blood half-life of the antibody, the pH-dependent binding capacity with FcRn needs to be improved.

Meanwhile, the action of antibodies as current pharmaceuticals has a direct method by binding to an antigen to inhibit the action of the antigen, and an indirect antigen removal method by effector cells (natural killer cells, macrophages, etc.) having an Fc gamma region and an Fc gamma receptor (FCGR) of the antibody binding to the antigen. In the case of an antibody therapeutic agent that is being developed recently, the effect of the drug is increased through an ADCC mechanism in which the effector cell recognizes the Fc gamma region of the antibody to attack and remove the antigen while preventing the action of the antigen due to binding to the antigen. In a recent ADCC-related study, it has been found that the binding capacity of the Fc gamma and Fc gamma receptor is affected depending on a genotype of the Fc gamma region receptor. Many studies have reported that the efficiency of the antibody-based therapeutic agent varies depending on the diversity of amino acid sequences of amino acid at position 131 of FCGR2A protein (FcyRIIIa) and amino acid at position 158 of FCGR3A protein, which are Fc gamma receptors of a patient. For example, in the case of Herceptin (Trastuzumab), which is a breast cancer therapeutic agent, there was reported in 2008 by Musolino, et al. a study that antibody-dependent cytotoxicity was increased with a significant difference in FCGR2A 131 H/H or FCGR3A 158 V/V genotype in preclinical studies. However, the binding capacity to a specific Fc receptor is increased by modifying an Fc region of a mammalian antibody, but the binding capacity to other Fc receptors is also maintained, so that there is a problem that undesirable immune responses are still maintained. Four of five major FcyRs in humans induce immuno-activating or inflammatory responses, and FcyRIIb induces immuno-inhibitory or anti-inflammatory responses. Most naturally occurring antibody or recombinant glycosylated antibodies bind to activating and inhibitory Fc receptors. The immune cells that most strongly induce the cancer cell death effect of therapeutic IgG antibodies through ADCC are natural killer cells (NK cells), and the NK cells express FcyRIIIa on the surface, but do not express FcyRI and FcyRIIa, FcyRIIb, and FcyRIIIb unlike other immune cells (e.g., monocytes, macrophages, dendritic cells, etc.), and thus, in order to maximize the cancer cell death mechanism, it is required to improve the affinity to FcyRIIIa expressed on the surface of the NK cells through optimization of the Fc region of the IgG antibody. In addition, considering the complex immune response of not only NK cells but also various immune cells present in the blood, the higher a ratio (A/I ratio) of the ability (A) of the Fc domain of an antibody to bind to activating FcyR and the ability (I) to bind to inhibitory FcyRIIb, the higher the ADCC induction ability, and thus, it is very urgent to selectively increase the binding capacity to the activating receptor compared to the binding capacity to FcyRIIb, which is an inhibitory receptor (Boruchov et al, J Clin Invest, 115(10):2914-23, 2005).

### [Disclosure]

### [Technical Problem]

An object of the present invention is to provide a novel human antibody Fc domain variant.

In addition, another object of the present invention is to provide an antibody specific to an Fc gamma receptor or an immunologically active fragment thereof.

In addition, yet another object of the present invention is to provide a nucleic acid molecule encoding the Fc domain variant, or the antibody or immunologically active fragment thereof, a vector including the nucleic acid molecule, and a host cell including the vector.

In addition, yet another object of the present invention is to provide a bioactive polypeptide conjugate with increased in vivo half-life.

In addition, still another object of the present invention is to provide a pharmaceutical composition for treating or preventing cancer.

In addition, still another object of the present invention is to provide a method for preparing a human antibody Fc domain variant.

In addition, still another object of the present invention is to provide a method for preparing an antibody specific to an Fc gamma receptor.

In addition, still another object of the present invention is to provide a use of an antibody or an immunologically active fragment thereof to be used for preparation of an antibody therapeutic agent.

In addition, still another object of the present invention is to provide a use of an antibody or an immunologically active fragment thereof for the prevention or treatment of cancer.

In addition, still another object of the present invention is to provide a method for treating cancer including administering an antibody or an immunologically active fragment thereof in a pharmaceutically effective amount to a subject suffering from cancer.

### [Technical Solution]

An aspect of the present invention provides a novel human antibody Fc domain variant with increased in vivo half-life and increased selective binding capacity to a specific Fc gamma receptor.

In addition, another aspect of the present invention provides an antibody or an immunologically active fragment thereof including the novel human antibody Fc domain variant.

In addition, yet another aspect of the present invention provides a bioactive polypeptide conjugate including the novel human antibody Fc domain variant.

In addition, yet another aspect of the present invention provides a nucleic acid molecule encoding the Fc domain variant, or the antibody or immunologically active fragment thereof, a vector including the nucleic acid molecule, and a host cell including the vector.

In addition, still another aspect of the present invention provides a pharmaceutical composition for preventing of treating cancer including the Fc domain variant, the bioactive polypeptide conjugate, or the antibody or immunologically active fragment thereof as an active ingredient.

In addition, still another aspect of the present invention provides a method for preparing a human antibody Fc domain variant.

In addition, still another aspect of the present invention provides a method for preparing an antibody specific to an Fc gamma receptor.

In addition, still another aspect of the present invention provides a use of the antibody or immunologically active fragment thereof of the present invention for use in the preparation of an antibody therapeutic agent.

In addition, still another aspect of the present invention provides a use of the antibody or immunologically active fragment thereof of the present invention for the prevention or treatment of cancer.

In addition, still another aspect of the present invention provides a method for treating cancer including administering the antibody or immunologically active fragment thereof of the present invention in a pharmaceutically effective amount to a subject suffering from cancer.

### [Advantageous Effects]

According to the present invention, novel human antibody Fc domain variants have lower capacity to bind to immune-inhibiting receptor FcyRIIb and have higher capacity to bind to immune activating receptor FcyRIIIa (increased A/I ratio) than a wild-type human antibody Fc domain and conventional antibodies approved as antibody therapeutic agents, thereby having remarkably improved ADCC induction ability and having maximized half-life in blood in which excellent pH-selective FcRn binding and unbinding capacity is exhibited, and thus bind to numerous peptide drug therapeutics having a low half-life and retention time in the body so as to enable the peptide drug therapeutics to have an increased blood half-life and exhibit long-term drug efficacy, and can maximize the immune mechanism of therapeutic protein drugs so as to be effectively used as an improved antibody drug.

### [Description of Drawings]

FIG. 1 is a diagram illustrating results of SDS-PAGE analysis after expression and purification of hFcγRIIIa-158V-streptavidin-His, hFcyRIIb-streptavidin-His, hFcγRIIIa-158V-GST, hFcγRIIIa-158F-GST, hFcγRIIb-GST and hFcRn-GST.
FIG. 2 is a schematic diagram illustrating the construction of a yeast display library for screening Fc variants with improved pH-dependent FcRn binding capacity and FcyRIIIa binding selectivity.
FIG. 3 is a diagram illustrating the concentration and gating strategy of Alexa647 conjugated tetrameric FcyRIIIa, non-fluorescent tetrameric FcyRIIb, and Alexa488 conjugated Protein A for each round of sorting for the yeast display library using FACS.
FIG. 4 is a diagram illustrating results of FACS analysis of (A) fluorescence intensities by FcγRIIIa-Alexa647 binding of each round of Fc sub-library screened through a Saccharomyces cerevisiae cell wall display, and (B) FcγRIIIa-Alexa647 binding fluorescence intensities in a state masked by non-fluorescent FcyRIIb (i.e., selective binding intensity to FcyRIIIa).
FIG. 5 is a diagram illustrating results of SDS-PAGE analysis after expression and purification of Trastuzumab Fc variants including Fc variants.
FIG. 6 illustrates results of ELISA analysis of FcRn binding capacity of Trastuzumab Fc variants including Fc variants at pH 6.0 and 7.4.
FIG. 7 is a diagram illustrating ELISA analysis of binding capacity to FcyRs (hFcγRIIIa-158V, hFcγRIIIa-158F and hFcγRIIb) of Trastuzumab Fc variants including Fc variants of MFc2, MFc3, MFc5 and MFc6 of the present invention.
FIG. 8 illustrates results of measuring quantitative binding capacity to pFcyRIIIa of Trastuzumab-Fc variants through Biolayer Interferometry (BLI) analysis.
FIG. 9 is a diagram illustrating results of SDS-PAGE analysis after expression and purification of Trastuzumab Fc variants including Fc variants introduced with novel mutations.
FIG. 10 is a diagram illustrating ELISA analysis of the binding capacity to FcyRs of Trastuzumab Fc variants including Fc variants introduced with novel mutations.

### [Best Mode of the Invention]

Hereinafter, embodiments of the present invention will be described in detail with reference to the accompanying drawings. However, the following embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Further, terminologies used in the present specification are terminologies used to properly express preferred embodiments of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present invention, unless otherwise defined, have the meaning as commonly understood by those skilled in the related art of the present invention. In addition, although preferred methods and samples are described in the present specification, similar or equivalent methods and samples thereto are also included in the scope of the present invention. The contents of all publications disclosed as references in this specification are incorporated in the present invention.

Throughout the present specification, general one-letter or three-letter codes for naturally existing amino acids are used, and generally allowed three-letter codes for other amino acids, such as α-aminoisobutyric acid (Aib) and N-methylglycine (Sar) are also used. The amino acids mentioned in the present invention as abbreviations are also described as follows according to the IUPAC-IUB nomenclature.

Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

In an aspect, the present invention relates to a human antibody Fc domain variant in which amino acids selected from the group consisting of amino acids at positions 235, 239, 248, 259, 268, 270, 298, 309, 311, 330, 332, 356, 378 and 428 numbered according to a Kabat numbering system in a wide-type human antibody Fc domain are substituted with sequences different from wild-type amino acids.

In one embodiment, the human antibody Fc domain variant of the present invention may include one or more amino acid substitutions selected from the group consisting of L235P, S239D, K248M, V259I, H268L, D270Y, S298G, L309E, Q311R, A330V, I332E, D356G, A378V and M428L.

In one embodiment, the human antibody Fc domain variant of the present invention may include an amino acid substitution of Q311M or Q311R, in which an amino acid at position 311 numbered according to the Kabat numbering system may be substituted with a sequence different from the wild-type amino acids in the wild-type human antibody Fc domain.

In one embodiment, the human antibody Fc domain variant of the present invention may be a human antibody Fc domain variant MFc2 including amino acid substitutions of L235P, V259I, Q311R, I332E, A378V and M428L, and the human antibody Fc domain variant MFc2 may include an amino acid sequence represented by SEQ ID NO: 1, which may be encoded by a nucleic acid molecule including a nucleotide sequence represented by SEQ ID NO: 2.

In one embodiment, the human antibody Fc domain variant of the present invention may be a human antibody Fc domain variant MFc3 including amino acid substitutions of S239D, H268L, Q311R, A330V, I332E and M428L, and the human antibody Fc domain variant MFc3 may include an amino acid sequence represented by SEQ ID NO: 3, which may be encoded by a nucleic acid molecule including a nucleotide sequence represented by SEQ ID NO: 4.

In one embodiment, the human antibody Fc domain variant of the present invention may be a human antibody Fc domain variant MFc5 including amino acid substitutions of S239D, K248M, H268L, L309E, Q311M, A330V, I332E and M428L, and the human antibody Fc domain variant MFc5 may include an amino acid sequence represented by SEQ ID NO: 5, which may be encoded by a nucleic acid molecule including a nucleotide sequence represented by SEQ ID NO: 6.

In one embodiment, the human antibody Fc domain variant of the present invention may be a human antibody Fc domain variant MFc6 including amino acid substitutions of L235P, S239D, D270Y, S298G, Q311R, I332E, D356G and M428L, and the human antibody Fc domain variant MFc6 may include an amino acid sequence represented by SEQ ID NO: 7, which may be encoded by a nucleic acid molecule including a nucleotide sequence represented by SEQ ID NO: 8.

In one embodiment, the wild-type human antibody Fc domain may consist of an amino acid sequence represented by SEQ ID NO: 9, which may be encoded with a nucleic acid molecule represented by SEQ ID NO: 10.

In one embodiment, the human antibody Fc domain variant of the present invention may have improved binding capacity to FcyRIIIa compared to the wild-type Fc domain.

In one embodiment, the human antibody Fc domain variant of the present invention has improved binding capacity to FcyRIIIa compared to the wild-type Fc domain, and has decreased binding capacity to FcyRIIb compared to the wild-type Fc domain, thereby selectively enhancing the binding to human FcyRIIIa compared to human FcyRIIb.

In one embodiment, the human antibody Fc domain variant of the present invention may enhance antibody dependent cell-mediated cytotoxicity (ADCC) induction ability compared to the wild-type human antibody Fc domain.

In one embodiment, the human antibody Fc domain variant of the present invention may exhibit low binding affinity to FcRn at pH 7.0 to 7.8 compared to the wild-type human antibody Fc domain, and may be pH sensitive that exhibits high binding affinity to FcRn at pH 5.6 to 6.5 compared to the wild-type human antibody Fc domain.

In one embodiment, the Fc variant of the present invention may exhibit high binding affinity to FcRn compared to the wild-type immunoglobulin Fc region at pH 5.6 to 6.5, and may be under weak acidic conditions in endosomes, and pH 5.8 to 6.0. The pH-sensitive Fc variant of the present invention may have increased binding affinity to FcRn in the pH range compared to the wild-type Fc domain of 10% or more, 20% or more, 30% or more, 40% or more, 50% or more, 60% or more, 70% or more, 80% or more, 90% or more, or 100% or more, or 2 times or more, 3 times or more, 4 times or more, 5 times or more, 6 times or more, 7 times or more, 8 times or more, 9 times or more, 10 times or more, 20 times or more, 30 times or more, 40 times or more, 50 times or more, 60 times or more, 70 times or more, 80 times or more, 90 times or more, or 100 times or more compared to the wild-type Fc domain.

In one embodiment, the Fc variant of the present invention may exhibit low binding affinity to FcRn compared to the wild-type immunoglobulin Fc region at pH 7.0 to 7.8, and may be under a normal pH range of blood and pH 7.2 to 7.6. The degree of unbinding (dissociation) of the Fc variant of the present invention from FcRn in the pH range may be the same or may not be substantially changed compared to the wild-type Fc domain.

In one embodiment, the human antibody Fc domain variant of the present invention may have increased in vivo half-life compared to the wild-type human antibody Fc domain.

In one embodiment, the half-life of the human antibody Fc domain variant of the present invention may be increased at least 10%, at least 20%, at least 30%, at least 40%, at least 50%, at least 60%, at least 70%, at least 80%, at least 90%, or at least 100% compared to the wild-type human antibody Fc domain, or increased at least 2 times, at least 3 times, at least 4 times, at least 5 times, at least 6 times, at least 7 times, at least 8 times, at least 9 times or at least 10 times compared to the wild-type Fc domain.

In one embodiment, the human antibody (immunoglobulin) may be IgA, IgM, IgE, IgD or IgG, or modifications thereof, and may be IgG1, IgG2, IgG3 or IgG4, preferably an anti-HER2 antibody, and more preferably Trastuzumab. Papain degradation of the antibody forms two Fab domains and one Fc domain, and in a human IgG molecule, the Fc region is generated by papain degradation of the N-terminus at Cys 226 (Deisenhofer, Biochemistry 20: 2361-2370, 1981).

In the present invention, variants including amino acid mutations in the human antibody Fc region of the present invention are defined according to the amino acid modifications constituting an Fc region of a parent antibody, and conventional antibody numbering is followed by the EU index by Kabat (Kabat et al., Sequence of proteins of immunological interest, 5th Ed., United States Public Health Service, National Institutes of Health, Bethesda, 1991).

As used in the present invention, the term "Fc domain variant" may be used interchangeably with the "Fc variant".

As used in the present invention, the term "wild-type polypeptide" refers to an unmodified polypeptide that is modified later to produce a derivative. The wild-type polypeptide may be a polypeptide found in nature, or a derivative or manipulation of the polypeptide found in nature. The wild-type polypeptide may refer to a polypeptide itself, a composition including the wild-type polypeptide, or an amino acid sequence encoding the same. Accordingly, as used in the present invention, the term "wild-type antibody" refers to an unmodified antibody polypeptide in which amino acid residues are modified to produce a derivative. Interchangeably with the term, the "parent antibody" may be used to refer to an unmodified antibody polypeptide into which amino acid modifications are introduced to produce a derivative.

As used in the present invention, the term "amino acid modification/mutation" refers to substitution, insertion and/or deletion, preferably substitution of amino acids in a polypeptide sequence. As used in the present invention, the term "amino acid substitution" or "substitution" means that an amino acid at a specific position in the polypeptide sequence of the wild-type human antibody Fc domain is replaced with another amino acid. For example, an Fc variant including Q311R substitution means that glutamine, an amino acid residue at position 311 in the amino acid sequence of the wild-type antibody Fc domain, is replaced with arginine.

As used in the present invention, the term "Fc variant" means including a modification of one or more amino acid residues compared to the wild-type antibody Fc domain.

The Fc variant of the present invention includes one or more amino acid modifications compared to the wild-type antibody Fc domain (region or fragment), resulting in a difference in amino acid sequence. The amino acid sequence of the Fc variant according to the present invention is substantially homologous to the amino acid sequence of the wild-type antibody Fc domain. For example, the amino acid sequence of the Fc variant according to the present invention has about 80% or more, preferably about 90% or more, and most preferably about 95% or more homology compared to the amino acid sequence of the wild-type antibody Fc domain. The amino acid modifications may be performed genetically using molecular biological methods, or also performed using enzymatic or chemical methods.

The Fc variants of the present invention may be prepared by any method known in the art. In one embodiment, the human antibody Fc variant according to the present invention is used to form a nucleic acid in which a polypeptide sequence including a specific amino acid modification is encoded and then, if desired, cloned into a host cell, expressed, and assayed. Various methods therefor are described in the literature (Molecular Cloning-A Laboratory Manual, 3rd Ed., Maniatis, Cold Spring Harbor Laboratory Press, New York, 2001; Current Protocols in Molecular Biology, John Wiley & Sons).

The nucleic acid encoding the Fc variant according to the present invention may be inserted into an expression vector for protein expression. The expression vector generally includes a protein operably linked, i.e., functionally related to a control or regulatory sequence, a selectable marker, an optional fusion partner, and/or an additional element. Under appropriate conditions, the Fc variant according to the present invention may be produced by a method of inducing the protein expression by culturing a host cell transformed with nucleic acid, preferably a nucleic acid-containing expression vector encoding the Fc variant according to the present invention. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, the Fc variant according to the present invention is produced using E. coli, which has low production cost and high industrial value, as a host cell.

Accordingly, the scope of the present invention includes a method for preparing an Fc variant including culturing a host cell into which a nucleic acid encoding an Fc variant has been introduced, under conditions suitable for protein expression; and purifying or isolating the Fc variant expressed from the host cell.

As used in the present invention, the term "FcRn" or "neonatal Fc receptor" refers to a protein that binds to an IgG antibody Fc region, which is at least partially encoded by an FcRn gene. The FcRn may be derived from any organism, including humans, mice, rats, rabbits, and monkeys, but is not limited thereto. As known in the art, a functional FcRn protein includes two polypeptides, often referred to as light and heavy chains. The light chain is β-2-microglobulin, and the heavy chain is encoded by the FcRn gene. Unless otherwise stated in the present specification, the FcRn or one FcRn protein refers to a complex of the FcRn heavy chain and β-2-microglobulin.

In an aspect, the present invention relates to an antibody specific to an Fc gamma receptor including the Fc domain variant of the present invention or an immunologically active fragment thereof.

In one embodiment, the antibody of the present invention may have increased in vivo half-life compared to the wild-type human antibody.

In one embodiment, the antibody may be a polyclonal antibody, a monoclonal antibody, a minibody, a domain antibody, a bispecific antibody, an antibody mimetic, a chimeric antibody, an antibody conjugate, a human antibody, or a humanized antibody. The immunologically active fragment may be Fab, Fd, Fab', dAb, F(ab'), F(ab')₂, a single chain fragment variable (scFv), Fv, a single chain antibody, an Fv dimer, a complementarity determining region fragment, or a diabody.

In one embodiment, the antibody including the Fc domain variant of the present invention, or the immunologically active fragment thereof may increase an effector action and has improved binding capacity to FcyRIIIa and decreased binding capacity to FcyRIIb compared to the wild-type Fc domain to have high FcyRIIIa binding selectivity and a high A/I ratio, thereby increasing antibody dependent cellular cytotoxicity (ADCC).

In the present invention, the A/I ratio is a ratio (A/I ratio) between the ability (A) to bind to activating FcyR and the ability (I) to bind to inhibitory FcyRIIb in the Fc domain of the antibody, and as the A/I ratio is increased, excellent ADCC induction ability is shown, so that it is important to selectively increase the binding capacity of the activating receptor compared to the binding capacity of FcyRIIb, which is an inhibiting receptor.

The antibody may be isolated or purified by various methods known in the art. A standard purification method includes chromatographic techniques, electrophoresis, immunology, precipitation, dialysis, filtration, concentration, and chromatofocusing techniques. As known in the art, various natural proteins such as bacterial proteins A, G, and L are bound to the antibody and these proteins may be used for purification. Often, purification by specific fusion partners may be possible.

The antibody includes functional fragments of an antibody molecule as well as a whole antibody form. The whole antibody has a structure having two full-length light chains and two full-length heavy chains, and each light chain is linked to the heavy chain by disulfide bonds. The functional fragment of the antibody molecule refers to a fragment having an antigen-binding function. Examples of the antibody fragment include (i) a Fab fragment consisting of a light chain variable region VL, a heavy chain variable region VH, a light chain constant region CL, and a first heavy chain constant region CH1; (ii) a Fd fragment consisting of VH and CH1 domains; (iii) an Fv fragment consisting of VL and VH domains of a single antibody; (iv) a dAb fragment consisting of a VH domain (Ward ES et al., Nature 341:544-546 (1989)); (v) an isolated CDR region; (vi) a F(ab')2 fragment, which is a bivalent fragment including two linked Fab fragments; (vii) a single-chain Fv molecule (scFv) in which the VH domain and the VL domain are linked to each other by a peptide linker to form an antigen-binding domain; (viii) a bispecific single-chain Fv dimer (PCT/US92/09965); and (ix) a diabody which is a multivalent or multispecific fragment produced by gene fusion (WO94/13804).

The antibody or the immunologically active fragment thereof of the present invention may be selected from the group consisting of animal-derived antibodies, chimeric antibodies, humanized antibodies, human antibodies, and immunologically active fragments thereof. The antibody may be produced recombinantly or synthetically.

The antibody or the immunologically active fragment thereof may be isolated from a living body (not present in a living body) or may non-naturally occur, for example, may be synthetically or recombinantly produced.

As used in the present invention, the "antibody" refers to a substance produced by stimulation of an antigen in the immune system, and the type thereof is not particularly limited, and may be obtained naturally or non-naturally (e.g., synthetically or recombinantly). The antibody is advantageous for mass expression and production because of being very stable in vivo as well as ex vivo and having a long half-life. In addition, since the antibody essentially has a dimer structure, avidity is very high. An intact antibody has a structure having two full-length light chains and two full-length heavy chains, and each light chain is linked to the heavy chain by disulfide bonds. The constant region of the antibody is divided into a heavy chain constant region and a light chain constant region, and the heavy chain constant region has gamma (γ), mu (µ), alpha (α), delta (δ), and epsilon (ε) types, and subclasses include gamma 1 (γ1), gamma 2 (γ2), gamma 3 (γ3), gamma 4 (γ4), alpha 1 (α1) and alpha 2 (α2). The light chain constant region has kappa (κ) and lambda (λ) types.

As used in the present invention, the term "heavy chain" is interpreted as meaning including all of a full-length heavy chain including a variable region domain V_{H} including an amino acid sequence having a variable region sequence enough to impart specificity to an antigen, three constant region domains C_{H}1, C_{H}2 and C_{H}3 and hinges, and fragments thereof. In addition, the term "light chain" is interpreted as meaning including all of a full-length light chain including a variable region domain V_{L} including an amino acid sequence having a variable region sequence enough to impart specificity to an antigen and a constant region domain C_{L}, and fragments thereof.

As used in the present invention, the term "Fc domain", "Fc fragment", or "Fc region" forms an antibody with a Fab domain/fragment, and the Fab domain/fragment consists of a light chain variable region V_{L} and a heavy chain variable region V_{H}, a light chain constant region C_{L}, and a first heavy chain constant region C_{H}1, and the Fc domain/fragment consists of a second constant region C_{H}2 and a third constant region C_{H}3 of the heavy chain.

In an aspect, the present invention relates to a nucleic acid molecule encoding the Fc domain variant of the present invention, or an antibody including the Fc domain variant or an immunologically active fragment thereof.

In one embodiment, the nucleic acid molecule encoding the Fc variant according to the present invention may include a nucleotide sequence represented by SEQ ID NO: 2, 4, 6, or 8.

In an aspect, the present invention relates to a vector including the nucleic acid molecule and a host cell including the vector.

The nucleic acid molecule of the present invention may be isolated or recombinant, and includes not only DNA and RNA in single-stranded and double-stranded forms, but also complementary sequences corresponding thereto. The isolated nucleic acid is a nucleic acid that is isolated from surrounding genetic sequences present in the genome of a subject from which the nucleic acid is isolated, in the case of a nucleic acid isolated from a naturally occurring source. In the case of a nucleic acid synthesized enzymatically or chemically from a template, such as a PCR product, a cDNA molecule, or an oligonucleotide, the nucleic acid produced from such a procedure may be understood as an isolated nucleic acid molecule. The isolated nucleic acid molecule refers to a nucleic acid molecule in the form of a separate fragment or as a component of a larger nucleic acid construct. The nucleic acid is operably linked when disposed in a functional relationship with another nucleic acid sequence. For example, DNA of a pre-sequence or secretory leader is expressed as a preprotein in the form before the polypeptide is secreted to be operably linked to DNA of the polypeptide, and a promoter or enhancer is operably linked to a coding sequence when affecting the transcription of the polypeptide sequence, or a ribosome binding domain is operably linked to a coding sequence when disposed to promote the translation. In general, the operably linked means that DNA sequences to be linked are located contiguously, and that the secretory leader exists contiguously in the same leading frame. However, the enhancer needs not to be contiguously located. The linkage is achieved by ligation at a convenient restriction enzyme site. When there is no site, synthetic oligonucleotide adapters or linkers are used according to conventional methods.

In the isolated nucleic acid molecule encoding the Fc domain variants of the present invention, the antibody including the Fc domain variant or the immunologically active fragment thereof, due to the degeneracy of codons or in consideration of codons preferred in an organism in which the protein is to be expressed, it will be well understood by those skilled in the art that various modifications may be made to a coding region within a range without changing the amino acid sequence of the Fc domain variant expressed from the coding region, or the antibody including the Fc domain variant or the immunologically active fragment thereof to be expressed from the coding region, various modifications or changes may be made within a range without affecting the expression of the gene even in parts other than the coding region, and such modified genes are also included within the scope of the present invention. That is, as long as the nucleic acid molecule of the present invention encodes a protein having equivalent activity thereto, one or more nucleobases may be mutated by substitution, deletion, insertion, or a combination thereof, which are also included in the scope of the present invention. The sequence of such a nucleic acid molecule may be single- or double-stranded, and may be a DNA molecule or an RNA (mRNA) molecule.

The isolated nucleic acid molecule encoding the Fc domain variant of the present invention, or the antibody including the Fc domain variant or the immunologically active fragment thereof may be inserted into an expression vector for protein expression. The expression vector generally includes a protein operably linked, i.e., functionally related to a control or regulatory sequence, a selectable marker, an optional fusion partner, and/or an additional element. In appropriate conditions, the Fc domain variant of the present invention, or the antibody including the Fc domain variant or the immunologically active fragment thereof may be produced by a method for inducing the protein expression by culturing a host cell transformed with a nucleic acid, preferably an expression vector including an isolated nucleic acid molecule encoding the Fc domain variant of the present invention, the antibody including the Fc domain variant or the immunologically active fragment thereof. A variety of suitable host cells including mammalian cells, bacteria, insect cells, and yeast may be used, but are not limited thereto. Methods for introducing exogenous nucleic acid into a host cell are known in the art and will vary depending on a host cell to be used. Preferably, it is possible to produce E. coli, which has high industrial value due to low production cost, as a host cell.

The vector of the present invention may include a plasmid vector, a cosmid vector, a bacteriophage vector, a viral vector, etc., but is not limited thereto. The suitable vector includes a signal sequence or a leader sequence for membrane targeting or secretion in addition to expression regulatory elements such as a promoter, an operator, an initiation codon, a termination codon, a polyadenylation signal, and an enhancer and may be variously produced according to a purpose. The promoter of the vector may be constitutive or inductive. The signal sequence may use a PhoA signal sequence, an OmpA signal sequence, etc. in the case of Escherichia sp. bacteria as a host, an α-amylase signal sequence, a subtilisin signal sequence, etc. in the case of Bacillus sp. bacteria as a host, an MFα signal sequence, a SUC2 signal sequence, etc. in the case of yeast as a host, and an insulin signal sequence, an α-interferon signal sequence, an antibody molecule signal sequence, etc. in the case of an animal cell as a host, but is not limited thereto. Further, the vector may include a selective marker for selecting a host cell including a vector and a replicable expression vector includes a replication origin.

As used in the present invention, the term "vector" refers to a vehicle into which a nucleic acid sequence may be inserted for introduction into a cell capable of replicating the nucleic acid sequence. The nucleic acid sequence may be exogenous or heterologous. The vector may include plasmids, cosmids, and viruses (e.g., bacteriophages), but is not limited thereto. Those skilled in the art may construct vectors by standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994 etc.).

In one embodiment, when constructing the vector, expression regulatory sequences such as a promoter, a terminator, and an enhancer, sequences for membrane targeting or secretion, etc. are appropriately selected according to a type of host cell to produce to produce the Fc domain variant, or the antibody including the Fc domain variant or the immunologically active fragment thereof and may be variously combined depending on a purpose.

As used in the present invention, the term "expression vector" refers to a vector including a nucleic acid sequence encoding at least a portion of a gene product to be transcribed. In some cases, the RNA molecule is then translated into a protein, a polypeptide, or a peptide. The expression vector may include various regulatory sequences. In addition to regulatory sequences that regulate transcription and translation, vectors and expression vectors may also include nucleic acid sequences that provide other functions.

As used in the present invention, the term "host cell" includes eukaryotes and prokaryotes, and refers to any transformable organism capable of replicating the vector or expressing a gene encoded by the vector. The host cell may be transfected or transformed by the vector, which means a process in which an exogenous nucleic acid molecule is delivered or introduced into the host cell.

In one embodiment, the host cell may be bacteria or animal cells, the animal cell line may be a CHO cell, a HEK cell or a NSO cell, and the bacteria may be E. coli.

In one aspect, the present invention relates to a bioactive polypeptide conjugate having an increased in vivo half-life by binding the human antibody Fc domain variant of the present invention and a bioactive polypeptide.

In one embodiment, the bioactive polypeptide may be selected from the group consisting of human growth hormone, growth hormone-releasing hormone, growth hormone-releasing peptide, interferon, colony-stimulating factor, interleukin, interleukin soluble receptor, TNF soluble receptor, glucocerebrosidase, macrophage activator, macrophage peptide, B-cell factor, T-cell factor, protein A, allergy suppressor, necrosis glycoprotein, immunotoxin, lymphotoxin, tumor necrosis factor, tumor suppressor, metastasis growth factor, alpha-1 antitrypsin, albumin, apolipoprotein-E, erythropoietin, hyperglycosylated erythropoietin, blood factor VII, blood factor VIII, blood factor IX, plasminogen activator, urokinase, streptokinase, protein C, C-reactive protein, renin inhibitor, collagenase inhibitor, superoxide dismutase, leptin, platelet-derived growth factor, epidermal growth factor, osteogenic growth factor, bone formation-promoting protein, calcitonin, insulin, insulin derivatives, glucagon, glucagon like peptide-1, atriopeptin, cartilage-inducing factor, connective tissue activator, follicle-stimulating hormone, luteinizing hormone, follicle-stimulating hormone-releasing hormone, nerve growth factor, parathyroid hormone, relaxin, secretin, somatomedin, insulin-like growth factor, corticosteroid, cholecystokinin, pancreatic polypeptide, gastrin-releasing peptide, corticotropin-releasing factor, thyroid-stimulating hormone, receptors, receptor antagonist, cell surface antigen, monoclonal antibody, polyclonal antibody, antibody fragments, and virus-derived vaccine antigens, and anything that needs to increase blood half-life may be used without any particular restrictions.

In one embodiment, the human antibody Fc domain variant of the present invention may be usefully used as a carrier to increase the in vivo half-life of a bioactive polypeptide such as a protein drug, and the bioactive polypeptide conjugate including the human antibody Fc domain variant may be used as a sustained drug formulation with a significantly increased in vivo half-life.

In one embodiment, the human antibody Fc domain variant and the bioactive polypeptide of the present invention may be linked by a non-peptide polymer, and the non-peptide polymer usable herein may be selected from the group consisting of polyethylene glycol, polypropylene glycol, copolymer of ethylene glycol and propylene glycol, polyoxyethylated polyol, polyvinyl alcohol, polysaccharide, dextran, polyvinyl ethyl ether, biodegradable polymer such as polylactic acid (PLA) and polylactic-glycolic acid (PLGA), lipid polymer, chitin, hyaluronic acid, and combinations thereof, preferably polyethylene glycol. Derivatives thereof which are already known in the art and derivatives which may be easily prepared in a technical level of the art are included in the scope of the present invention.

In an embodiment, an antibody drug may be bound to the bioactive polypeptide conjugate including the human antibody Fc domain variant of the present invention, and the antibody drug for cancer therapy may be trastuzumab, cetuximab, bevacizumab, rituximab, basiliximab, infliximab, ipilimumab, pembrolizumab, nivolumab, atezolizumab, or avelumab.

In the antibody therapeutic agent, a mechanism for collecting and delivering immune cells to a target antigen is one of the most important mechanisms, and since the Fc domain of the antibody plays a critical role in the collection of immune cells and antibody-dependent cell-mediated cytotoxicity (ADCC), the Fc variant with increased selective binding to the Fc gamma receptor of the present invention is advantageous to be used as an antibody for treatment. In particular, since the ADCC function of the antibody depends on interaction with the Fc gamma receptors (FcyR) present on the surfaces of many cells, and a type of immune cell to be collected is determined depending on which Fc receptor among five Fc receptors in humans the antibody binds to, attempts to modify the antibody so as to collect a specific cell are very important in the therapeutic field.

The present invention includes a method for preparing a sustained drug formulation by covalently linking the human antibody Fc domain variant of the present invention to a bioactive polypeptide through a non-peptide polymer.

The preparation method according to the present invention may include covalently linking a bioactive polypeptide and a human antibody Fc domain variant through a non-peptide polymer having a reactive group at a terminal; and isolating a conjugate in which the bioactive polypeptide, the non-peptide polymer, and the human antibody Fc domain variant are covalently linked.

In an aspect, the present invention relates to a pharmaceutical composition for preventing or treating cancer including the human antibody Fc domain variant of the present invention, the antibody including the Fc domain variant or the immunologically active fragment thereof, or the bioactive polypeptide conjugate including the antibody as an active ingredient.

In one embodiment, the cancer may be any one selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem glioma and pituitary adenoma.

In an embodiment, the antibody including the Fc domain variant of the present invention or the immunologically active fragment thereof has high FcyRIIIa binding selectivity to have a high A/I ratio, thereby increasing an effector function through natural killer cells (NK cells) and increasing antibody dependent cellular cytotoxicity (ADCC). Since the natural killer cells (NK cells), which have the strongest cancer cell killing effect, unlike other immune cells (e.g., monocytes, macrophages, dendritic cells), express FcyRIIIa on the surface, but do not express FcyRI, FcyRIIa, FcyRIIb, and FcyRIIIb, an antibody including the Fc domain variant with FcyRIIIa binding selectivity of the present invention or an immunologically active fragment thereof may maximize a cancer cell killing mechanism through NK cells.

In one embodiment, the composition of the present invention may further include an immunogenic cell death inducing agent. The immunogenic cell death inducing agent may be any one or more selected from the group consisting of anthracycline-based anticancer agents, taxane-based anticancer agents, anti-EGFR antibodies, BK channel agonists, bortezomib, cardiac glycoside, cyclophosmide-based anticancer agents, GADD34/PP1 inhibitors, LV-tSMAC, Measles virus, bleomycin, mitoxantrone, or oxaliplatin. The anthracycline-based anticancer agent may be daunorubicin, doxorubicin, epirubicin, idarubicin, pixantrone, sabarubicin, or valrubicin, and the taxane-based anticancer agent may be paclitaxel or docetaxel.

The pharmaceutical composition for preventing or treating cancer of the present invention is administered together with a chemical anticancer drug (anticancer agent) and the like to increase a cancer therapy effect of conventional anticancer agents through the death effect of cancer cells. Combined administration may be performed simultaneously or sequentially with the anticancer agent. Examples of the anticancer agent include DNA alkylating agents such as mechloethamine, chlorambucil, phenylalanine, mustard, cyclophosphamide, ifosfamide, carmustine (BCNU), lomustine (CCNU), streptozotocin, busulfan, thiotepa, cisplatin, and carboplatin; anticancer antibiotics such as dactinomycin (actinomycin D), plicamycin, and mitomycin C; plant alkaloids such as vincristine, vinblastine, etoposide, teniposide, topotecan, and iridotecan, and the like, but are not limited thereto.

As used in the present invention, the term "prevention" means all actions that inhibit or delay the occurrence, spread, and recurrence of cancer by administration of the pharmaceutical composition according to the present invention.

As used in the present invention, the term "treatment" refers to all actions that improve or beneficially change the death of cancer cells or the symptoms of cancer by administration of the composition of the present invention. Those skilled in the art to which the present invention pertains will be able to determine the degree of improvement, enhancement and treatment by knowing the exact criteria of a disease for which the composition of the present invention is effective by referring to data presented by the Korean Academy of Medical Sciences, etc.

As used in the present invention, the term "therapeutically effective amount" used in combination with the active ingredient means an amount of pharmaceutically acceptable salt of the composition effective for preventing or treating a target disease, and the therapeutically effective amount of the composition of the present invention may vary depending on many factors, such as a method of administration, a target site, the condition of a patient, and the like. Accordingly, when used in the human body, the dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective amount. As used in the present invention, the term "pharmaceutically effective amount" refers to an amount enough to treat the disease at a reasonable benefit/risk ratio applicable to medical treatment and enough not to cause side effects. The effective dose level may be determined according to factors including the health condition of a patient, the type and severity of cancer, the activity of a drug, the sensitivity to a drug, a method of administration, a time of administration, a route of administration, an excretion rate, duration of treatment, and combination or simultaneously used drugs, and other factors well-known in the medical field. The composition of the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may further include a pharmaceutically acceptable additive. At this time, the pharmaceutically acceptable additive may use starch, gelatinized starch, microcrystalline cellulose, lactose, povidone, colloidal silicon dioxide, calcium hydrogen phosphate, lactose, mannitol, syrup, arabic gum, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc and the like. The pharmaceutically acceptable additive according to the present invention is preferably included in an amount of 0.1 part by weight to 90 parts by weight based on the composition, but is not limited thereto.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present invention may be administered parenterally (e.g., applied with injectable formulations intravenously, subcutaneously, intraperitoneally or topically) or orally according to a desired method, and the range of the dose may vary depending on the body weight, age, sex, and health condition of a patient, a diet, an administration time, an administration method, an excretion rate, the severity of a disease, and the like. A daily dose of the composition according to the present invention is 0.0001 to 10 mg/ml, preferably 0.0001 to 5 mg/ml, and more preferably administered once to several times a day.

Liquid formulations for oral administration of the composition of the present invention correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In an aspect, the present invention relates to a method for preparing a human antibody Fc domain variant including a) culturing a host cell including a vector including a nucleic acid molecule encoding the human antibody Fc domain variant of the present invention; and b) recovering a polypeptide expressed by the host cell.

In an aspect, the present invention relates to a method for preparing an antibody specific to an Fc gamma receptor including a) culturing a host cell including a vector including a nucleic acid molecule encoding the antibody or immunologically active fragment thereof of the present invention; and b) purifying the antibody expressed by the host cell.

In one embodiment, the purification of the antibody may include filtration, HPLC, anion exchange or cation exchange, high performance liquid chromatography (HPLC), affinity chromatography, or a combination thereof, preferably affinity chromatography using Protein A.

In an aspect, the present invention provides a use of an antibody including the Fc domain variant of the present invention or an immunologically active fragment thereof for use in the preparation of an antibody therapeutic agent.

In an aspect, the present invention relates to a use of an antibody including the Fc domain variant of the present invention or an immunologically active fragment thereof for prevention or treatment of cancer.

In an aspect, the present invention relates to a method for treating cancer including administering an antibody including the Fc domain variant of the present invention or an immunologically active fragment thereof in a pharmaceutically effective amount to a subject suffering from cancer.

### [Mode of the Invention]

Hereinafter, the present invention will be described in more detail with reference to the following Examples. However, the following Examples are only intended to embody the contents of the present invention, and the present invention is not limited thereto.

### Example 1. Expression and purification of target substances

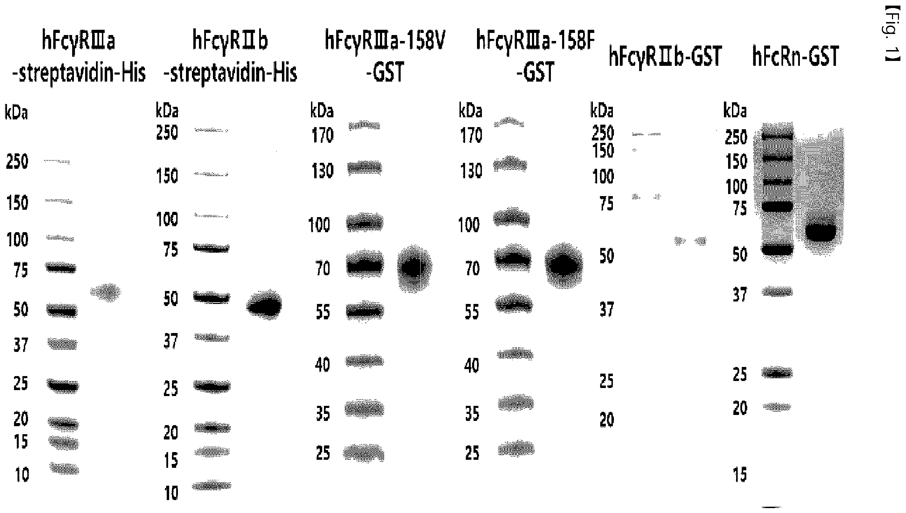

To discover Fc variants with improved selective binding to FcyRIIIa and to analyze binding capacity to FcyRs and FcRn of Fc variants, target substances were expressed and purified. Specifically, six types of expression vectors of pMAZ-hFcγRIIIa-158V-streptavidin-His, pMAZ-hFcγRIIb-streptavidin-His, pMAZ-hFcγRIIIa-158V-GST, pMAZ-hFcγRIIIa-158F-GST, pMAZ-hFcyRIIb-GST and pMAZ-hFcRn-GST were prepared. Polyethylenimine (PEI, Polyscience, 23966) and the expression vector genes were mixed in 30 ml of a Freestyle 293 expression culture medium (Gibco, 12338-018) at a ratio of 4 : 1, incubated at room temperature for 20 minutes, and then transfected into Expi293F animal cells cultured at a density of 2 × 10⁶ cells/ml and cultured for 7 days under conditions of 37°C, 125 rpm, and 8% CO₂. After culturing, the supernatant was collected by centrifugation at 6000×g for 15 minutes, and 12.5 ml of 25×PBS was mixed with 30 ml of the culture supernatant and filtered using a 0.2 µm bottle top filter (Corning, 430513). 500 µl of Ni-NTA resin (QIAGEN, 40724) was added to a culture medium transfected with each of the filtered pMAZ-hFcγRIIIa-158V-streptavidin-His and pMAZ-hFcγRIIb-streptavidin-His, stirred for 16 hours at 4°C, and then packed in a disposable polypropylene column to recover the resin. The resin was washed sequentially with 100 ml of PBS (pH 7.4), 25 ml of 10 mM imidazole buffer diluted in PBS, and 25 ml of 20 mM imidazole buffer diluted in PBS, and eluted with 250 mM imidazole buffer diluted in 4 ml of PBS. In addition, 500 µl of GST resin (Incospharm, 1101-3) was added to the culture medium transfected with each of the filtered pMAZ-hFcγRIIIa-158V-GST, pMAZ-hFcγRIIIa-158F-GST, pMAZ-hFcyRIIb-GST, and pMAZ-hFcRn-GST, stirred at 4°C for 16 hours, and then packed in a disposable polypropylene column to recover the resin. The resin was washed with 100 ml 1×PBS (pH 7.4) and eluted with 50 mM Tris-HCl and 10 mM GSH buffer (pH 8.0) diluted in 4 ml of 1×PBS. Thereafter, the buffer was exchanged with 1×PBS (pH 7.4) using Amicon Ultra-4 centrifugal filter units 3K (Merck Millipore, UFC800324), and as a result, it was confirmed through SDS-PAGE gel that high purity of hFcγRIIIa-158V-streptavidin-His, hFcyRIIb-streptavidin-His, hFcγRIIIa-158V-GST, hFcγRIIIa-158F-GST, hFcyRIIb-GST and hFcRn-GST were successfully purified (FIG. 1). Among these, the purified hFcγRIIIa-158V-streptavidin-His prepared Alexa647 conjugated tetrameric FcyRIIIa using the Alexa Fluor^{™} 647 Protein Labeling Kit (Imvitrogen, A20173).

### Example 2. Construction of yeast display library for screening Fc variants with improved pH-dependent FcRn binding capacity and FcγRIIIa binding selectivity

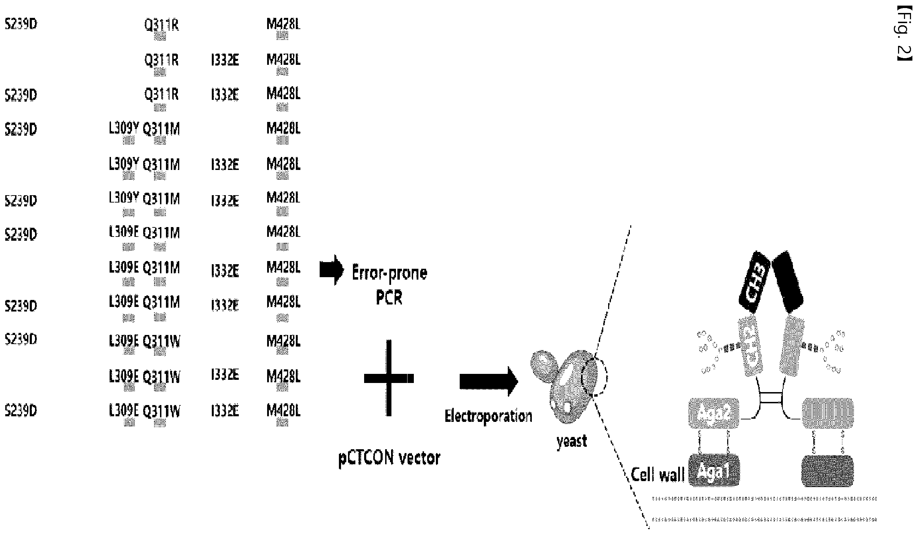

To efficiently select Fc variants with improved pH-dependent FcRn binding capacity and selective binding to FcyRIIIa, a total of 12 templates were prepared by introducing mutations S239D, I332E, and S239D/I332E capable of improving FcyRIIIa binding capacity to an antibody Fc sequence introduced with Q311R/M428L, L309Y/Q311M/M428L, L309E/Q311M/M428L, and L309E/Q311W/M428L having improved pH-dependent binding to FcRn, respectively, and through error-prone PCR, a DNA library was prepared in which mutations were introduced with a probability of 0.68% among the entire Fc sequence. A yeast display library with diversity of a total of 5.5 × 10⁷ antibody Fcs was constructed by transferring a total of 24 µg of the constructed DNA library to 800 µl of yeast sp. Saccharomyces cerevisiae competent cells through electroporation using a MicroPulser Electroporator (Bio-Rad, #1652100) along with 8 µg of a pCTCON vector encoding a Aga2 protein, a yeast cell wall anchoring protein for yeast display and a transformation selection marker gene (Trp1) and then culturing the cells in 500 ml of a tryptophan-free SD medium [Difco Yeast nitrogen base (BD, 291940) 6.7 g/l, Bacto casamino acid (BD, 223050) 5.0 g/l, Na₂HPO₄ (JUNSEI,7558-79-4) 5.4 g/l, NaH₂PO₄.H₂O (SAMCHUN, 10049-21-5) 8.56 g/l and Glucose (DUKSAN, 50-99-7) 20 g/l] to selectively survive only the transformed yeast sp. (FIG. 2).

### Example 3. Discovery of Fc variants with improved selective binding capacity to FcγRIIIa

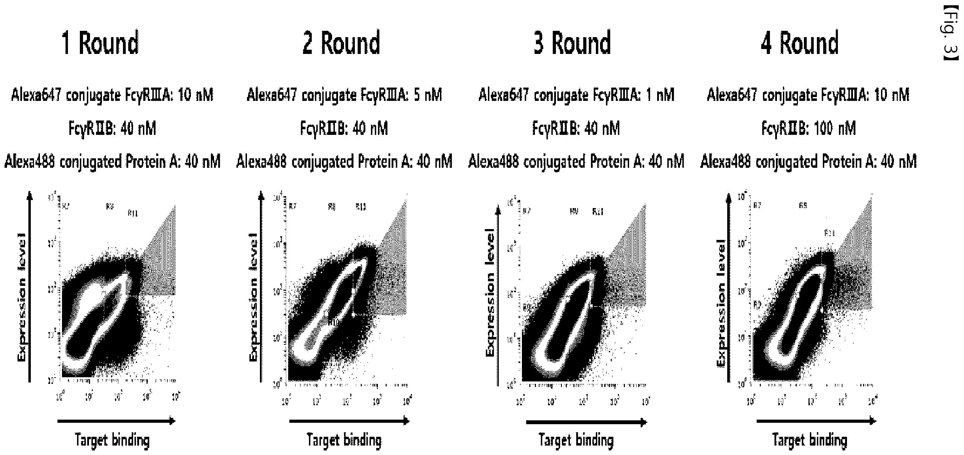

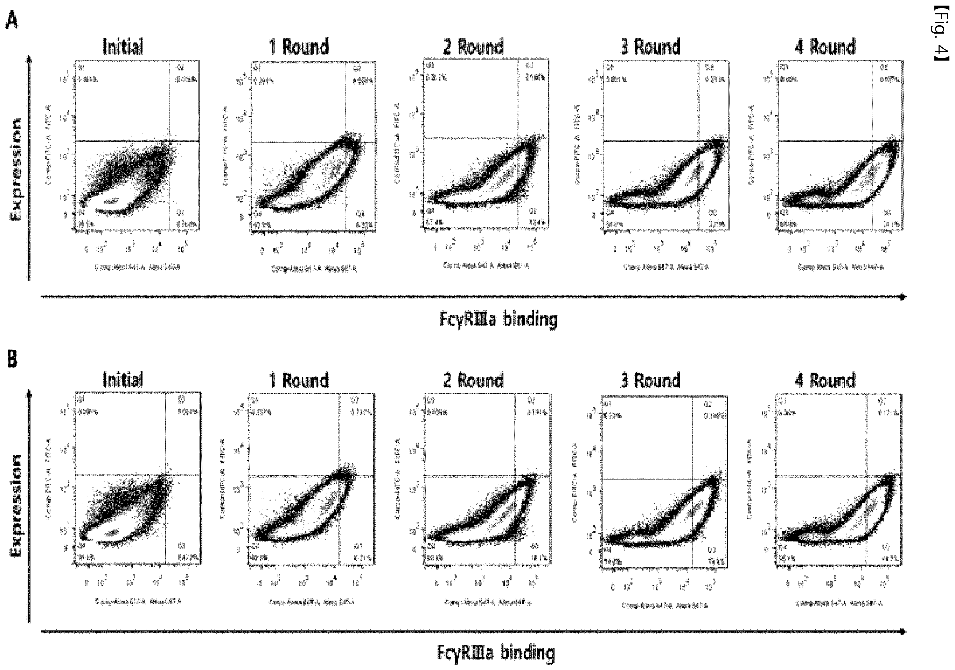

The expression levels of the displayed Fc library were improved by culturing 5.5 × 10⁸ cells of the constructed yeast library in 100 ml of the tryptophan-free SD medium for 16 hours at 30°C, and then culturing 7 × 10⁸ cells in 100 ml of a tryptophan-free SG medium [Difco Yeast nitrogen base (BD, 291940) 6.7 g/l, Bacto casamino acid (BD, 223050) 5.0 g/l, Na₂HPO₄ (JUNSEI,7558-79-4) 5.4 g/l, NaH₂PO₄.H₂O (SAMCHUN, 10049-21-5) 8.56 g/l and Galactose (Sigma-Aldrich, 59-23-4) 20 g/1] for 48 hours at 20°C. Among the cultured cells, 1 × 10⁸ cells were centrifuged at 14,000 × g for 30 seconds at 4°C, and the supernatant was removed, and then washed with 1 ml PBSB (1×PBS, 0.1% bovine serum albumin (gibco, 30063-572)) (pH 7.4) and then 40 nM Alexa488 conjugated Protein A, 10 nM Alexa647 conjugated tetrameric FcyRIIIa, and 40 nM non-fluorescent tetrameric FcyRIIb were incubated in 1 ml of diluted PBSB (pH 7.4) for 1 hour. After incubation, the cells were washed with 1 ml PBSB (pH 7.4) and resuspended in 1 ml PBSB (pH 7.4) to induce competitive binding of Alexa488-conjugated Protein A, Alexa647-conjugated tetrameric FcyRIIIa, and non-fluorescent FcyRIIb, and then cells showing high fluorescence signals were recovered through a flow cytometer (Bio-Rad S3 sorter, Bio-Rad, #1451029). In the recovered sub-library, the concentration of Alexa647-conjugated tetrameric FcyRIIIa was decreased or the concentration of non-fluorescent tetrameric FcyRIIb was increased using the same method, and through a total of 4 rounds of sorting, cells displaying Fc variants with excellent selective binding capacity to FcyRIIIa were enriched (FIGS. 3 and 4). Through random selection from the last enriched sub-library, finally, a total of four Fc variants MFc2 (L235P/V259I/Q311R/I332E/A378V/M428L), MFc3 (S239D/H268L/Q311R/A330V/I332E/M428L), MFc5 (S239D/K248M/H268L/L309E/Q311M/A330V/I332E/M428L) and MFc6 (L235P/S239D/D270Y/S298G/Q311R/I332E/D356G/M428L) with improved pH-dependent FcRn binding capacity and FcγRIIIa/FcγRIIb selective binding were secured (FIG. 4 and Table 1).

**[Table 1]**

| Variant name | Introduced mutation |
|---|---|
| MFc2 | L235P/V259I/Q311R/I332E/A378V/M428L |
| MFc3 | S239D/H268L/Q311R/A330V/I332E/M428L |
| MFc5 | S239D/K248M/H268L/L309E/Q311M/A330V/I332E/M428L |
| MFc6 | L235P/S239D/D270Y/S298G/Q311R/I332E/D356G/M428L |

### Example 4. Expression and purification of Trastuzumab Fc variants with improved FcyRIIIa selective binding capacity

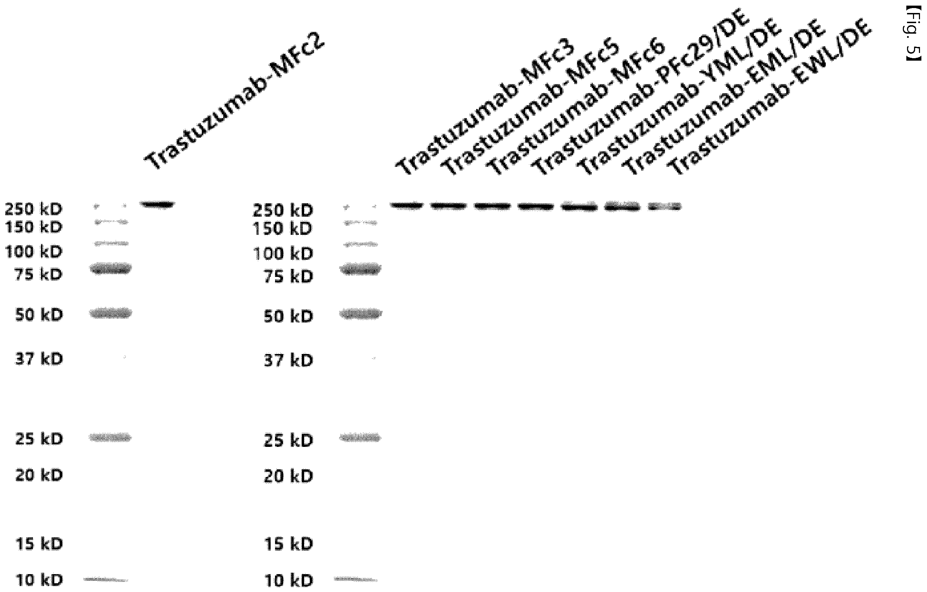

In order to express and purify Trastuzumab Fc variants including the three Fc variants selected in Example 3, respectively, four types of expression vectors pMAZ-Trastuzumab-HC-Pfc29/DE (PFc29/DE: S239D/Q311R/I332E/M428L), pMAZ-Trastuzumab-HC-YML/DE (YML/DE: S239D/L309Y/Q311M/I332E/M428L), pMAZ-Trastuzumab-HC-EML/DE (EML/DE: S239D/L309E/Q311M/I332E/M428L) and pMAZ-Trastuzumab-HC- EWL/DE (EWL/DE: S239D/L309E/Q311W/I332E/M428L) introduced into the Fc region of the Trastuzumab antibody were prepared by combining four types of expression vectors for Trastuzumab Fc variants pMAZ-Trastuzumab-HC-MFc2, pMAZ-Trastuzumab-HC-MFc3, pMAZ-Trastuzumab-HC-MFc5, and pMAZ-Trastuzumab-HC-MFc6, mutations of existing FcRn variants with improved pH-dependent binding used as templates for library construction, and mutations of DE variants with improved FcyRIIIa binding. Heavy chain genes and light chain genes of the respective variants were first mixed in 3 ml of a Freestyle 293 expression culture medium (Gibco, 12338-018) at a 1 : 1 ratio, and mixed at a ratio of PEI : variant gene = 4:1, left at room temperature for 20 minutes, and then transfected into Expi293F cells subcultured the previous day at a density of 2 × 10⁶ cells/ml. Thereafter, the transfected cells were incubated for 7 days under conditions of 37°C, 125 rpm, and 8% CO₂ in a CO₂ shaking incubator, and centrifuged at 6000 ×g for 15 minutes to take only the supernatant. Thereafter, 30 ml of the taken culture supernatant and 1.25 ml of 25×PBS were mixed and then filtered using a 0.2 µm bottle top filter (Corning, 430513). 100 µl of Protein A resin was added to the filtered culture medium, stirred at 4°C for 16 hours, and then packed in a disposable polypropylene column to recover the resin. The resin was washed with 5 ml of 1×PBS (pH 7.4), eluted with 3 ml of 100 mM glycine (pH 2.7) buffer, and then neutralized using 1 M Tris-HCl (pH 8.0). The buffer was exchanged with 1×PBS (pH 7.4) using Amicon Ultra-4 centrifugal filter units 3K (Merck Millipore, UFC800324), and it was confirmed through SDS-PAGE gel that the antibody Trastuzumab-Fc variants were successfully purified with high purity (FIG. 5).

### Example 5. Analysis of FcRn binding capacity of Trastuzumab-Fc variants

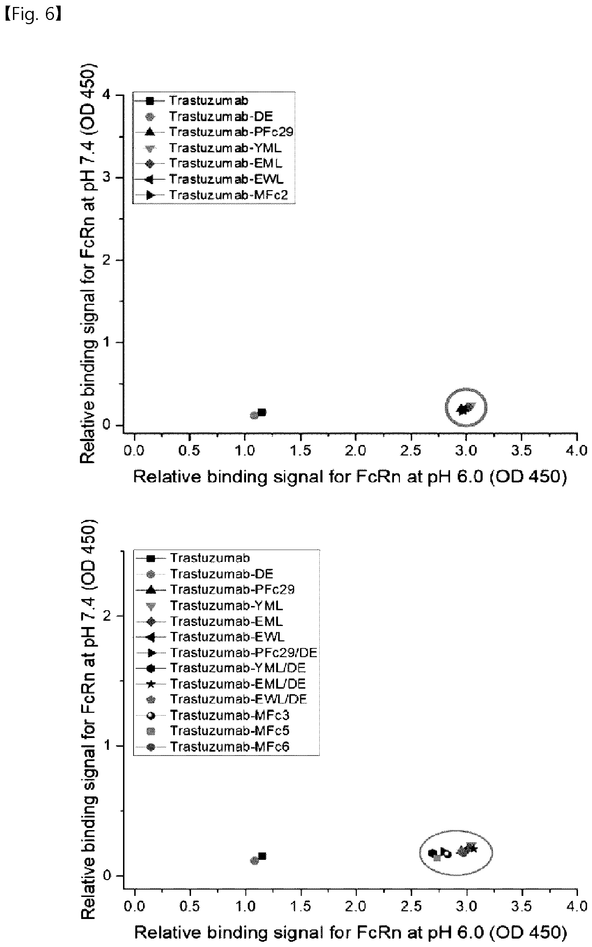

The pH-dependent human FcRn binding capacity of Trastuzumab-Fc variants, including the Fc variants MFc2, MFc3, MFc5, and MFc6 expressed and purified in Example 4, respectively, was analyzed using ELISA. Specifically, 50 µl each of HER2 diluted in 4 µg/ml of 0.05 M Na₂CO₃ (pH 9.6) was incubated and immobilized in a flat bottom polystyrene High Bind 96 well microplate (Costar, 3590) for 16 hours at 4°C, and then blocked for 2 hours at room temperature with 4% skim milk (GenomicBase, SKI400) diluted in 100 µl of 1×PBS (pH 7.4). After washed 4 times with 150 µl of 0.05% PBST (pH 7.4), 50 µl of Trastuzumab Fc variants diluted to 20 µg/ml in 1% skim milk diluted in 1×PBS (pH 7.4) were dispensed into each well and reacted at room temperature for 1 hour. After washed 4 times with 100 µl of 0.05% PBST (pH 6.0/pH 7.4), 50 µl of hFcRn-GST diluted to a concentration of 1 µg/ml in 1% skim milk diluted in 1×PBS (pH 6.0/pH 7.4) were dispensed into each well and reacted at room temperature for 1 hour. After washed four times with 100 µl of 0.05% PBST, the antibody reaction was performed for 1 hour at room temperature using 50 µl of anti-GST-HRP conjugate (GE Healthcare, RPN1236V) diluted in 1% skim milk (pH 6.0/pH 7.4) diluted in 1×PBS and washed four times with 100 µl of 0.05% PBST (pH 6.0/pH 7.4). 50 µl each of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek). As a result, it was confirmed that all of the four types of Trastuzumab variants (Trastuzumab-Fc variants) including the Fc variants of the present invention showed significantly improved hFcRn-dependent binding at pH 6.0 to pH 7.4 compared to Trastuzumab-Fc variants into which wild-type Trastuzumab or a DE (S239D/I332E) variant was introduced (FIG. 6). In addition, it was confirmed that all of the four types of Trastuzumab variants showed significantly improved hFcRn pH-dependent binding at pH 6.0 to pH 7.4 compared to Trastuzumab-Fc variants into which PFc29 (Q311R/M428L), YML (L309Y/Q311M/M428L), EML (L309E/Q311M/M428L), EWL (L309E/Q311W/M428L), PFc29/DE, YML/DE, EML/DE and EWL/DE variants developed by the present inventors were introduced, respectively (FIG. 6).

### Example 6. Analysis of binding capacity to FcγRs of Trastuzumab-Fc variants

ELISA analysis was performed to confirm the FcyRs binding capacity of Trastuzumab-Fc variants, including Fc variants MFc2, MFc3, MFc5 and MFc6, respectively. Specifically, 50 µl each of HER2 diluted to 4 µg/ml in 0.05 M Na₂CO₃ (pH 9.6) was incubated and immobilized in a flat bottom polystyrene High Bind 96 well microplate (Costar, 3590) for 16 hours at 4°C, and then blocked for 2 hours at room temperature with 4% skim milk (GenomicBase, SKI400) diluted in 100 µl of 1×PBS (pH 7.4). After washed 4 times with 150 µl of 0.05% PBST (1×PBS, 0.05% Tween 20 (Sigma-Aldrich, P1379-1L)) (pH 7.4), 50 µl of Trastuzumab-Fc variants diluted to 20 µg/ml in 1% skim milk diluted in 1×PBS (pH 7.4) were dispensed into each well and reacted at room temperature for 1 hour. After washed four times with 100 µl of 0.05% PBST, 50 µl of hFcyRs-GST (hFcγRIIIa-158V-GST, hFcγRIIIa-158F-GST or hFcyRIIb-GST) diluted to a concentration of 1 µg/ml with 1% skim milk diluted in 1×PBS (pH 7.4) was dispensed into each well and reacted at room temperature for 1 hour. After washed 4 times with 100 µl of 0.05% PBST (pH 7.4), antibody reaction was performed for 1 hour at room temperature using 50 µl of anti-GST-HRP conjugate (GE Healthcare, RPN1236V) and washed four times with 100 µl of 0.05% PBST (pH 7.4). 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek).

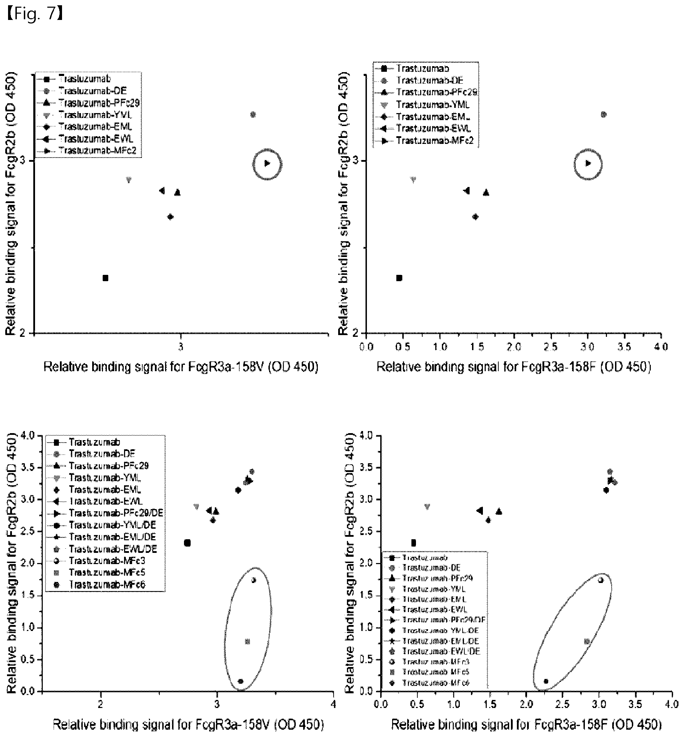

As a result, it was confirmed that all four types of Trastuzumab-Fc variants including the Fc variants MFc2, MFc3, MFc5, and MFc6 of the present invention, respectively, had significantly improved both the binding capacity to hFcγRIIIa-158V and the binding capacity to hFcγRIIIa-158F compared to Trastuzumab-Fc variants introduced with PFc29, YML, EML, and EWL variants, respectively, and wild-type Trastuzumab. It was confirmed that all of the four types of Trastuzumab-Fc variants of the present invention had significantly improved binding capacity to hFcγRIIIa-158V and binding capacity to hFcγRIIIa-158F compared the Trastuzumab-Fc variants introduced with existing DE, PFc29/DE, YML/DE, EML/DE, and EWL/DE variants of the present inventors, respectively (FIG. 7). All of the four types of Trastuzumab-Fc variants of the present invention showed significantly reduced FcyRIIb binding capacity compared to DE and wild-type Trastuzumab, even when comparing DE and PFc29, which were known to have significantly improved binding capacity to FcyRIIb, a receptor of inhibiting immune cell activity compared to wild-type Trastuzumab, and Trastuzumab-Fc variants introduced with PFc29/DE, YML/DE, EML/DE and EWL/DE variants, which combined DE with Fc variants PFc29, YML, EML and EWL having improved pH-dependent FcRn binding capacity previously developed by the present inventor (FIG. 7). Therefore, it was confirmed that all of the four Fc variants obtained in the present invention had significantly improved selective binding to FcyRIIIa compared to FcyRIIb, compared to DE, PFc29, YML, EML and EWL variants.

### Example 7. Analysis of binding capacity to FcyRIIIa of Trastuzumab-Fc variants

In order to determine the quantitative binding capacity to FcyRIIIa of four types of Trastuzumab variants, including Fc variants of the present invention of MFc2 (L235P/V259I/Q311R/I332E/A378V/M428L), MFc3 (S239D/H268L/Q311R/A330V/I332E/M428L), MFc5 (S239D/K248M/H268L/L309E/Q311M/A330V/I332E/M428L), and MFc6 (L235P/S239D/D270Y/S298G/Q311R/I332E/D356G/M428L), respectively, Biolayer interferometry (BLI) analysis was performed using Octet R8 (Sartorius) instrument and Octet Ni-NTA (NTA) Biosensors (Sartorius, 18-5101). Specifically, 200 µl of deionized water was added to wells of a 96-well plate, and hydrated for 10 minutes by inserting a biosensor. Then, in the well of a 96-well plate, hFcγRIIIa-158V-streptavidin-His or hFcγRIIIa-158F-streptavidin-His diluted to a concentration of 4 µg/ml in 200 µl of deionized water and 1×PBS (pH 7.4), and a control group or Trastuzumab-Fc variant serially diluted two-fold at 1,000 nM to 15.6 nM were sequentially dispensed into each row to form a sample plate, and then the biosensor and the sample plate were added in the Octet R8 instrument, and the biosensor reacted with deionized water for 60 seconds (baseline), hFcyRIIIa for 300 seconds (loading), 1×PBS (pH 7.4) for 120 seconds (baseline), Trastuzumab-Fc variant for 90 seconds (association), and 1×PBS (pH 7.4) for 150 seconds (dissociation) to measure the binding capacity.

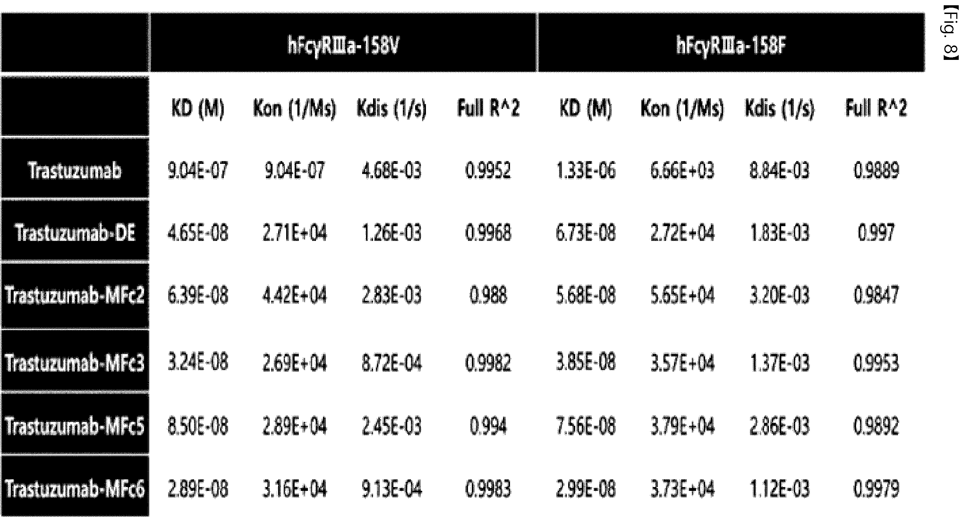

As a result, it was confirmed that the Trastuzumab Fc variants (Trastuzumab-MFc2, Trastuzumab-MFc3, Trastuzumab-MFc5 and Trastuzumab-MFc6) of the present invention had significantly improved binding affinity to hFcγRIIIa-158V and hFcγRIIIa-158Fdp compared to Trastuzumab having wild-type Fc, like Trastuzumab-DE (S239D/I332E) from Xencor. Particularly, it was confirmed that the binding capacity to hFcγRIIIa-158V and hFcγRIIIa-158Fdp of the trastuzumab-MFc2 variant was improved 14.1 and 23.4 times compared to DE from Xencor, respectively, and Trastuzumab-MFc3 or Trastuzumab-MFc6 had significantly improved binding capacity to hFcγRIIIa-158V and hFcγRIIIa-158F compared to DE from Xencor (FIG. 8).

### Example 8. Analysis of causative mutations of improved pH-dependent FcRn binding capacity and FcγRIIIa selective binding

### 8-1. Expression and purification of Trastuzumab-Fc variants introduced with novel mutations alone

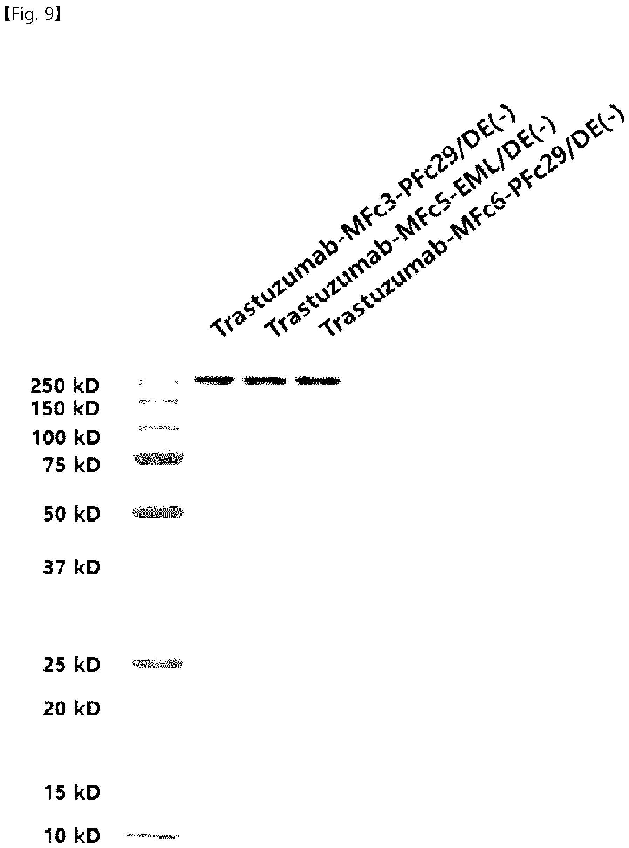

To confirm whether the significantly improved pH-dependent FcRn binding characteristics and FcyRIIIa selective binding characteristics, compared to FcyRIIb, of the four types of Fc variants of the present invention were caused by conventional pH-dependent FcRn binding improved mutations and FcyRIIIa binding improved mutations (DE), three types of expression vectors introduced with novel mutations alone of pMAZ-Trastuzumab-HC-MFc3-PFc29/DE(-) (MFc3-PFc29/DE(-): H268L/A330V), pMAZ-Trastuzumab-HC-MFc5-EML/DE(-) (MFc5-EML/DE(-): K248M/H268L/A330V) and pMAZ-Trastuzumab-HC-MFc6-PFc29/DE(-) (MFc6-PFc29/DE(-): L235P/D270Y /S298G/D356G) were prepared by substituting again each of known mutations with improved pH-dependent FcRn binding and known DE variant mutations with improved FcyRIIIa binding with a wild type, respectively (Table 2), in the three types of Trastuzumab-Fc variants discovered in the present invention. Heavy chain genes and light chain genes of the respective variants were first mixed in 3 ml of a Freestyle 293 expression culture medium (Gibco, 12338-018) at a 1 : 1 ratio, and mixed at a ratio of PEI: variant gene = 4 : 1, left at room temperature for 20 minutes, and then transfected into Expi293F cells subcultured the previous day at a density of 2 × 10⁶ cells/ml. Thereafter, the transfected cells were incubated for 7 days under conditions of 37°C, 125 rpm, and 8% CO₂ in a CO₂ shaking incubator, and centrifuged at 6000 ×g for 15 minutes to take only the supernatant. Thereafter, 30 ml of the taken culture supernatant and 1.25 ml of 25×PBS were mixed and then filtered using a 0.2 µm bottle top filter (Corning, 430513). 100 µl of Protein A resin was added to the filtered culture medium, stirred at 4°C for 16 hours, and then packed in a disposable polypropylene column to recover the resin. The resin was washed with 5 ml of 1×PBS (pH 7.4), eluted with 3 ml of 100 mM glycine (pH 2.7) buffer, and then neutralized using 1 M Tris-HCl (pH 8.0). The buffer was exchanged with 1×PBS (pH 7.4) using Amicon Ultra-4 centrifugal filter units 3K (Merck Millipore, UFC800324), and it was confirmed through SDS-PAGE gel that the antibody Trastuzumab-Fc variants were successfully purified with high purity (FIG. 9).

**[Table 2]**

| Variant name | Introduced mutation |
|---|---|
| MFc3-PFc29/DE(-) | H268L/A330V |
| MFc5-EML/DE(-) | K248M/H268L/A330V |
| MFc6-PFc29/DE(-) | L235P/D270Y/S298G/D356G |

### 8-2. Confirmation of binding capacity to FcyRs

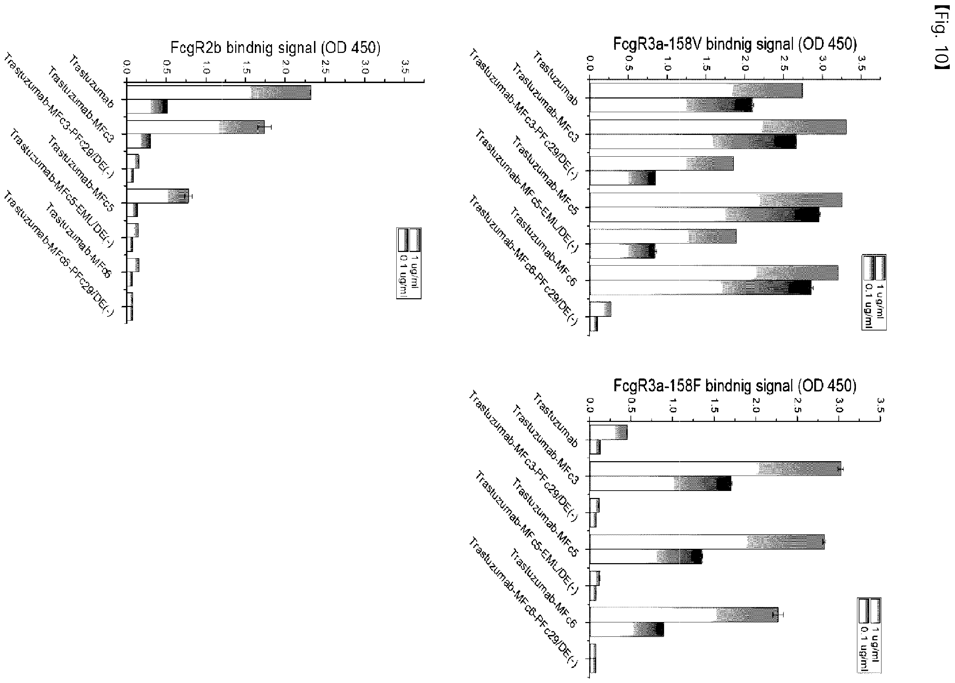

ELISA analysis was performed to confirm the binding capacity to FcyRs of Trastuzumab variants including Fc variants of MFc3 (S239D/H268L/Q311R/A330V/I332E/M428L), MFc5 (S239D/K248M/H268L/L309E/Q311M/A330V/I332E/M428L) and MFc6 (L235P/S239D/D270Y/S298G/Q311R/I332E/D356G/M428L) of the present invention and Trastuzumab variants including MFc3-PFc29/DE(-) (H268L/A330V), MFc5-EML/DE(-) (K248M/H268L/A330V) and MFc6-PFc29/DE(-) (L235P/D270Y/S298G/D356G) introduced with novel mutations alone. Specifically, 50 µl each of HER2 diluted to 4 µg/ml in 0.05 M Na₂CO₃ (pH 9.6) was incubated and immobilized in a flat bottom polystyrene High Bind 96 well microplate (Costar, 3590) for 16 hours at 4°C, and then blocked for 2 hours at room temperature with 4% skim milk (GenomicBase, SKI400) diluted in 100 µl of 1×PBS (pH 7.4). After washed 4 times with 150 µl of 0.05% PBST (1×PBS, 0.05% Tween 20 (Sigma-Aldrich, P1379-1L)) (pH 7.4), 50 µl of Trastuzumab-Fc variants diluted to 20 µg/ml in 1% skim milk diluted in 1×PBS (pH 7.4) were dispensed into each well and reacted at room temperature for 1 hour. After washed four times with 100 µl of 0.05% PBST, 50 µl of hFcyRs-GST (hFcγRIIIa-158V-GST, hFcγRIIIa-158F-GST or hFcγRIIb-GST) diluted to a concentration of 1 µg/ml or 0.1 µg/ml with 1% skim milk diluted in 1×PBS (pH 7.4) was dispensed into each well and reacted at room temperature for 1 hour. After washed 4 times with 100 µl of 0.05% PBST (pH 7.4), antibody reaction was performed for 1 hour at room temperature using 50 µl of anti-GST-HRP conjugate (GE Healthcare, RPN1236V) and washed four times with 100 µl of 0.05% PBST (pH 7.4). 50 µl of a 1-Step Ultra TMB-ELISA substrate solution (Thermo Fisher Scientific, 34028) was added to develop color, and then added with 50 µl each of 2 M H₂SO₄ to terminate the reaction. Absorbance was measured using an Epoch microplate spectrophotometer (BioTek). As a result, it was confirmed that all three types of Trastuzumab-Fc variants in which novel mutations alone were introduced had significantly decreased binding capacity to hFcγRIIIa-158V, hFcγRIIIa-158F and FcyRIIb compared to Trastuzumab-Fc variants of the present invention of Trastuzumab-MFc3, Trastuzumab-MFc5, and Trastuzumab-MFc6 (FIG. 10). Through this, it may be seen that the significantly improved pH-dependent binding characteristics for hFcRn and the selective binding characteristics for FcyRIIIa compared to FcyRIIb of the Fc variants of the present invention are formed by the combined action of the mutations introduced into each variant.

## Claims

1. A human antibody Fc domain variant in which amino acids at positions 235, 239, 248, 259, 268, 270, 298, 309, 311, 330, 332, 356, 378 and 428 numbered according to a Kabat numbering system in a wide-type human antibody Fc domain are substituted with sequences different from wild-type amino acids.

2. The human antibody Fc domain variant of claim 1, wherein the human antibody Fc domain variant comprises one or more amino acid substitutions selected from the group consisting of L235P, S239D, K248M, V259I, H268L, D270Y, S298G, L309E, Q311R, A330V, I332E, D356G, A378V and M428L.

3. The human antibody Fc domain variant of claim 1, wherein the human antibody Fc domain variant comprises amino acid substitutions of L235P, V259I, Q311R, I332E, A378V and M428L.

4. The human antibody Fc domain variant of claim 1, wherein the human antibody Fc domain variant comprises amino acid substitutions of S239D, H268L, Q311R, A330V, I332E and M428L.

5. The human antibody Fc domain variant of claim 1, wherein the human antibody Fc domain variant comprises amino acid substitutions of S239D, K248M, H268L, L309E, Q311M, A330V, I332E and M428L.

6. The human antibody Fc domain variant of claim 1, wherein the human antibody Fc domain variant comprises amino acid substitutions of L235P, S239D, D270Y, S298G, Q311R, I332E, D356G and M428L.

7. The human antibody Fc domain variant of claim 1, wherein the human antibody Fc domain variant has an enhanced selective binding capacity to human FcyRIIIa compared to human FcyRIIb.

8. The human antibody Fc domain variant of claim 1, wherein the human antibody Fc domain variant has enhanced antibody-dependent cell-mediated cytotoxicity (ADCC) induction ability compared to the wild-type human antibody Fc domain.

9. The human antibody Fc domain variant of claim 1, wherein the human antibody Fc domain variant exhibits low binding affinity to FcRn at pH 7.0 to 7.8 compared to the wild-type human antibody Fc domain.

10. The human antibody Fc domain variant of claim 1, wherein the human antibody Fc domain variant exhibits high binding affinity to FcRn at pH 5.6 to 6.5 compared to the wild-type human antibody Fc domain.

11. The human antibody Fc domain variant of claim 1, wherein the human antibody Fc domain variant has increased in vivo half-life compared to the wild-type human antibody Fc domain.

12. An antibody specific to an Fc gamma receptor comprising the Fc domain variant of claim 1, or an immunologically active fragment thereof.

13. The antibody or immunologically active fragment thereof of claim 12, wherein the antibody or immunologically active fragment thereof has increased in vivo half-life compared to the wild-type human antibody.

14. A bioactive polypeptide conjugate having increased in vivo half-life by binding the human antibody Fc domain variant of claim 1 and a bioactive polypeptide.

15. The bioactive polypeptide conjugate of claim 14, wherein the bioactive polypeptide is selected from the group consisting of human growth hormone, growth hormone-releasing hormone, growth hormone-releasing peptide, interferon, colony-stimulating factor, interleukin, interleukin soluble receptor, TNF soluble receptor, glucocerebrosidase, macrophage activator, macrophage peptide, B-cell factor, T-cell factor, protein A, allergy suppressor, necrosis glycoprotein, immunotoxin, lymphotoxin, tumor necrosis factor, tumor suppressor, metastasis growth factor, alpha-1 antitrypsin, albumin, apolipoprotein-E, erythropoietin, hyperglycosylated erythropoietin, blood factor VII, blood factor VIII, blood factor IX, plasminogen activator, urokinase, streptokinase, protein C, C-reactive protein, renin inhibitor, collagenase inhibitor, superoxide dismutase, leptin, platelet-derived growth factor, epidermal growth factor, osteogenic growth factor, bone formation-promoting protein, calcitonin, insulin, insulin derivatives, glucagon, glucagon like peptide-1, atriopeptin, cartilage-inducing factor, connective tissue activator, follicle-stimulating hormone, luteinizing hormone, follicle-stimulating hormone-releasing hormone, nerve growth factor, parathyroid hormone, relaxin, secretin, somatomedin, insulin-like growth factor, corticosteroid, cholecystokinin, pancreatic polypeptide, gastrin-releasing peptide, corticotropin-releasing factor, thyroid-stimulating hormone, receptors, receptor antagonist, cell surface antigen, monoclonal antibody, polyclonal antibody, antibody fragments, and virus-derived vaccine antigens.

16. A nucleic acid molecule encoding the human antibody Fc domain variant of claim 1, or the antibody or immunologically active fragment thereof of claim 12.

17. A vector comprising the nucleic acid molecule of claim 16.

18. A host cell comprising the vector of claim 17.

19. A pharmaceutical composition for preventing or treating cancer comprising the human antibody Fc domain variant of claim 1, the antibody or immunologically active fragment thereof of claim 12, or the bioactive polypeptide conjugate of claim 14 as an active ingredient.

20. The pharmaceutical composition for preventing or treating cancer of claim 19, wherein the cancer is any one selected from the group consisting of brain tumor, melanoma, myeloma, non-small cell lung cancer, oral cancer, liver cancer, stomach cancer, colon cancer, breast cancer, lung cancer, bone cancer, pancreatic cancer, skin cancer, head or neck cancer, cervical cancer, ovarian cancer, colorectal cancer, small intestine cancer, rectal cancer, fallopian tube carcinoma, perianal cancer, endometrial carcinoma, vaginal carcinoma, vulvar carcinoma, Hodgkin's disease, esophageal cancer, lymph adenocarcinoma, bladder cancer, gallbladder cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, kidney or ureter cancer, renal cell carcinoma, renal pelvic carcinoma, central nervous system tumor, primary central nervous system lymphoma, spinal cord tumor, brainstem gliomas and pituitary adenomas.

21. A method for preparing a human antibody Fc domain variant comprising:
a) culturing a host cell comprising a vector including a nucleic acid molecule encoding the human antibody Fc domain variant of claim 1; and
b) recovering a polypeptide expressed by the host cell.

22. A method for preparing an antibody specific to an Fc gamma receptor comprising:
a) culturing a host cell comprising a vector including a nucleic acid molecule encoding the antibody or immunologically active fragment thereof of claim 12; and
b) purifying the antibody expressed by the host cell.

23. A use of an antibody comprising the Fc domain variant of claim 1 or an immunologically active fragment thereof for use in the preparation of an antibody therapeutic agent.

24. A use of an antibody comprising the Fc domain variant of claim 1 or an immunologically active fragment thereof for the prevention or treatment of cancer.

25. A method for treating cancer comprising administering an antibody containing the Fc domain variant of claim 1 or an immunologically active fragment thereof in a pharmaceutically effective amount to a subject suffering from cancer.
